# EUROPÄISCHE PATENTSCHRIFT

(11) **EP 0 884 986 B1**
(45) Veröffentlichungstag und Bekanntmachung des Hinweises auf die Patenterteilung: **30.06.2004**
(21) Anmeldenummer: 97949870.6
(22) Anmeldetag: 28.10.1997
(51) Int. Cl.: A61F 2/06

(54) **STENT**
STENT
EXTENSEUR

(30) Priorität: 28.10.1996 DE 19645289; 10.12.1996 DE 19653709
(43) Veröffentlichungstag der Anmeldung: 23.12.1998
(73) Patentinhaber: BIOTRONIK Mess- und Therapiegeräte GmbH & Co Ingenieurbüro Berlin, 12359 Berlin (DE)
(72) Erfinder: KRANZ, Curt, D-10825 Berlin (DE)
(74) Vertreter: Christiansen, Henning, Dipl.-Ing.
(86) Internationale Anmeldenummer: PCT/DE1997/002569
(87) Internationale Veröffentlichungsnummer: WO 1998/018404

(56) Entgegenhaltungen:
- EP-A- 0 796 597
- WO-A-95/32757
- WO-A-96/26689
- WO-A-97/25937
- DE-C- 4 429 380

## Beschreibung

Die Erfindung betrifft einen Stent, insbesondere Koronarstent, als intraluminales Expansionselement, der im Oberbegriff des Anspruchs 1 genannten Art.

Aus den europäischen Patentschriften EP-B1 0 364 787 und EP-B1 335 341 ist ein aufweitbares intraluminales Element mit mindestens einem dünnwandigen, rohrförmigen Teil (nachfolgend als Stent bezeichnet) bekannt. Die Mantelfläche des Stents ist durchbrochen netzförmig ausgebildet und weist dabei Ausnehmungen auf, die durch sich geradlinig in axialer und in Umfangsrichtung erstreckende stegartigen .Elemente von geringer Materialstärke begrenzt sind. Die stegartigen Elemente bestehen aus der restlichen Rohrwandung, von der das Material im Bereich der Ausnehmungen entfernt wurde.

Derartige Stents werden in einem operativen Eingriff unter Einwirkung von innen nach außen gerichteten Kräften durch einen mit Druckgas beaufschlagten schlauchförmigen Dilator, expandiert. Der Stent behält dabei trotz Verformung seine Rohrform bei und weitet das durch Ablagerungen verengte Gefäß auf.

Die WO 96/26689 zeigt einen Stent, dessen endseitige Streben zur Herabsetzung eines Verformungswiderstandes verlängert sind.

Aus der nachveröffentlichte EP 0 796 597 ist ein Stent bekannt, der erste und zweite axiale Abschnitte aufweist, wobei mindestens einer der axialen Abschnitte derart ausgebildet ist, dass sein Widerstand gegen Verformung und Rückverformung herabgesetzt ist. Der Abschnitt mit dem erniedrigten Verformungswiderstand kann in einem Endbereich angeordnet werden und aus einem gegenüber den weiteren Abschnitten des Stents dünneren Material geformt sein.

Die bekannten Stents weisen den Nachteil auf, daß das Expandieren aufgrund der Verformung der sich axial erstreckenden stegartiges Elemente sich im Bereich des Stentendes nur in beschränktem Maße an das sich in diesem Bereich angrenzende Gewebe anpassen kann, da der Formänderung der einzelnen stegartigen Elemente der Stents relativ enge Grenzen gesetzt sind.

Ausgehend von den Mängeln des Standes der Technik liegt der Erfindung die Aufgabe zugrunde, einen expandierbaren Stent der eingangs genannten Gattung anzugeben, welcher möglichst sicher - und damit auch ohne die Gefahr eines durch Spannungsüberlastung bedingten Bruchs im Bereich der stegartiges Elemente, expandierbar ist. Es tritt hier ein stufenförmiger Übergang ein, der den Bluttransport behindert.

Die Aufgabe wird durch die kennzeichnenden Merkmale des Anspruchs 1 gelöst.

Die Erfindung schließt die technische Lehre ein, daß am Ende eines von einem Stent erweiterten Gefäßbereiches ein möglichst "fließender" Übergang mit einer variablen ausgleichenden Querschnittsänderung erfolgen soll.

Eine Materialschwächung erfolgt zum Ende eines Stents oder Stentsegments hin, um hier den Widerstand gegen Verformungen zu verringern.

Dies geschieht durch eine Querschnittsverringerung der stegartigen Bereiche zum Stentende hin. Diese Querschnittsverringerung wird bevorzugt unter Beibehaltung der radialen Abmessungen erzeugt, so daß der Stent aus einem rohrförmigen Querschnitt mittels eines Laser-Schneidwerkzeugs durch Herausschneiden der ausgesparten Bereiche erzeugt werden kann.

Um eine kontinuierliche Anpassung zu erhalten, erfolgt eine - insbesondere lineare - Zunahme der Schwächung zum Ende des Stents hin.

Bei dem erfindungsgemäßen Stent- oder Stentsegment erfolgt also zunächst eine Expansion durch einen Dilator. Die Tendenz zur Verformung wird hierbei wegen der verringerten Steifigkeit im Endbereich auch schon bei der Expansion relativ größer sein als im mittleren Bereich. Dies wirkt sich aber nur begrenzt aus, da der Ballon in seinen Endbereichen ebenfalls eine geringere Expansionswirkung zeigt. Im übrigen wirkt auch das nichtlineare Elastizitätsverhalten zu größeren Verformungen hin einer starken Expansion entgegen. Wird nun der Ballon entfernt kann beim nunmehrigen Rückverformen des Gefäßes der Endbereich des Stents dieser Bewegung stärker folgen als dessen zentraler Bereich, der ja gerade durch die erhaltene plastische Verformung das Gefäß offenhalten soll. Auf diese Weise entsteht ein kontinuierlicher Übergang des Gefäßes von seinem durch den eingefügten Stent erweiterten Bereich zum (stentlosen) Bereich hin. In jedem Fall werden scharfe geformte Kanten im Bereich des Stentendes vermieden. Die Kontur des gestützten Gefäßbereichs geht kontinuierlich ohne Stufung oder Kante in den nicht gestützten Bereich über.

Die Herabsetzung des Widerstands gegen Verformung kann auf mannigfache Weise erzielt werden. So kann beispielsweise im Endbereich ein entsprechend weniger steifes Material vorgesehen werden oder ein Material, das auf chemische Weise (Anätzen) in seiner Struktur geschwächt. In günstiger Weise wird jedoch meist die Herabsetzung des Widerstands gegen Verformung und Rückverformung durch eine Querschnittsverringerung, d.h. durch eine Verringerung der Materialdicke durch entsprechende Dimensionierung oder Abtrag (mechanisch oder Laserstrahlung) hervorgerufen ist.

Die Herabsetzung des Widerstands gegen Verformung und Rückverformung zum Ende des Stents oder Stentsegments hin, läßt sich damit mit kontinuierlicher - also nicht sprunghafter - Krümmungsänderung erzielen. Dies gilt insbesondere, wenn die Materialschwächung zum Ende des Stents oder Stentsegments hin gleichmäßig zunimmt.

Eine Querschnittsverminderung kann in tangentialer Richtung bereits im Entwurf bei der Festlegung der Formgebung der Stegbereiche im Verhältnis zu den verbleibenden Bereichen der Rohrwandung (Verbindungsbereiche) erfolgen, während eine Querschnittsverringerung durch Materialabtrag bevorzugt in radialer Richtung von außen nach innen vorgenommen wird.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine bevorzugte Ausführungsform der Erfindung in Seitenansicht,
Figuren 2 ein Detail der in Figur 1 dargestellten Ausführungsform im Bereich des Stentendes.

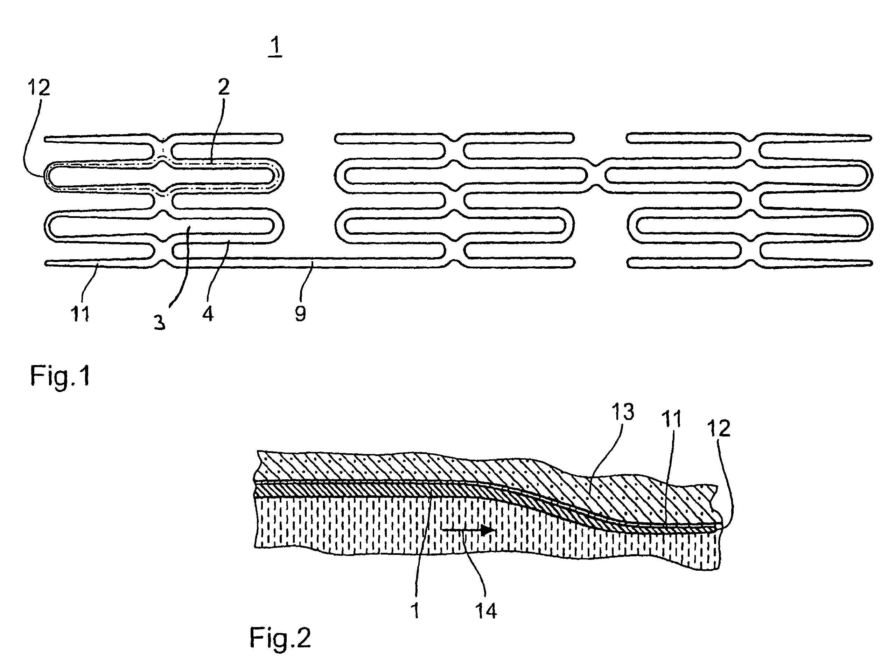

Der in Figur 1 wiedergegebene Stent 1 weist eine rohrförmig/hohlzylindrische Grundform mit zahlreichen Durchbrüchen auf, welche von expansiblen Strukturelementen umschlossen sind, die die Form von gestauchten Ringen aufweisen. Diese werden nachfolgend als "expansible Elemente" bezeichnet und sind an einem Beispiel mit einer strichpunktierten Linie 2 markiert. Diese expansiblen Elemente 2 werden gebildet durch umlaufende schmale stegartige Bereiche 4 mit rechtekkigem Querschnitt und zeichnen sich dadurch aus, daß sie eine Ausnehmung 3 ringförmig umschließen.

Die expansiblen Elemente 2 haben in vollständig expandiertem Zustand (in der Zeichnung nicht dargestellt) nahezu die Form eines Vielecks.

Die expansiblen Elemente 2 sind derart geformt, daß sie sich nach dem Einbringen des Stents in ein Gefäß durch Dilatieren mit einem Ballonkatheter mit minimaler Verformung in die Polygonalform umwandeln.

Die in Figur 1 dargestellte Ausführung eines Stents ist in mehrere, in axialer Richtung aufeinanderfolgende Segmente untergliedert. Diese Segmente sind unter sich gleichartig ausgebildet und weisen eine Mantelfläche auf, in welcher Ausnehmungen 3 in tangentialer Richtung aneinandergereiht und durch Verbindungsbereiche 9 miteinander verbunden sind.

Die vorstehend beschriebene Ausbildung der Stents 1 gestattet ein Expandieren der rohrförmigen Stents 1, ohne daß es an den Verbindungspunkten zur Ausbildung von zur Zerstörung von Stegbereichen führenden Extremwerten der Kerbspannung kommt.

In Figur 2 ist dargestellt, wie sich das Ende 11 des Stents 1 innerhalb eines Blutgefäßes durch abnehmende Steifigkeit kontinuierlich dem sich hier wieder verringernden Querschnitt des Gefäßes anpaßt. Der Bogenbereich 12 ist kaum mehr expandiert und ermöglicht ein verwirbelungsfreies Fließen des Blutstroms 14. Die Steifigkeit des Stents 1 ist also zu seinem Ende 11 hin so verringert, daß sich unter Berücksichtigung der Widerstandskraft des Blutgefäßes beim Expandieren ein kontinuierlicher Übergang ergibt.

Der hier dargestellte Stent 1 besteht aus Titan oder Tantal oder einem anderen biokompatiblen Material oder einer entsprechenden Metallegierung als Werkstoff, woraus eine gute Körperverträglichkeit und eine ausgezeichnete Verformbarkeit resultiert. Eine Mikrobeschichtung aus amorphem Siliciumcarbid wirkt einer Thrombenbildung entgegen.

## Patentansprüche

1. Stent (1), insbesondere Koronarstent, bestehend aus mindestens einem dünnwandigen, rohrförmigen Element, dessen Mantelfläche durchbrochen netzförmig ausgebildet ist und dabei Ausnehmungen (3) aufweist, die durch stegartige Elemente (4) von geringer Breite begrenzt sind, wobei die stegartigen Elemente (4) aus dem restlichen Material im Bereich der Ausnehmungen (3) entfernt wurden, und wobei zum Ende (11) eines Stents (1) hin ein Widerstand der stegartigen Elemente (4) oder der angrenzenden durch das restliche Material gebildeten Verbindungsbereiche gegen Verformung und Rückverformung im Vergleich zur übrigen Stent- oder Stentsegmentslänge derart herabgesetzt ist, dass seine Steifigkeit - und damit seine Fähigkeit expandierend zu wirken bzw. eine dem Gewebe erteilte Expansion aufrechtzuerhalten - verringert ist und die Herabsetzung des Widerstands gegen Verformung und Rückverformung durch Materialschwächung hervorgerufen ist, **dadurch gekennzeichnet, dass** die Herabsetzung des Widerstands gegen Verformung und Rückverformung zum Ende (11) des Stents (1) hin zunimmt.

2. Stent nach Anspruch 1, **dadurch gekennzeichnet, dass** die Materialschwächung zum Ende (11) des Stents (1) oder Stentsegments hin gleichmäßig zunimmt.

3. Stent nach Anspruch 1, **dadurch gekennzeichnet, dass** der Stent (1) aus Titan, Tantal oder einem anderen biokompatiblen Metall besteht.

4. Stent nach Anspruch 1, **dadurch gekennzeichnet, dass** eine Beschichtung aus amorphem Siliziumcarbid vorgesehen ist.

## Claims

1. A stent (1), in particular a coronary stent, consisting of at least one thin-walled, tubular element, the outer surface of which has an open, reticular construction and at the same time recesses (3), which are bounded by narrow web-like elements (4), wherein the web-like elements (4) were removed from the remaining material in the region of the recesses (3), and wherein towards the end (11) of a stent (1) the resistance to deformation and reverse deformation offered by the web-like elements (4) or the adjacent connecting areas formed by the remaining material is reduced in comparison to the remaining length of the stent or the stent segment in such a manner that its rigidity - and thus its ability to act in an expanding manner or to maintain the expansion imparted to the tissue - is reduced and the reduction in the resistance to deformation and reverse deformation is caused by material weakening,
**characterised in that** the reduction in the resistance to deformation and reverse deformation increases towards the end (11) of the stent (1).

2. A stent according to Claim 1,
**characterised in that** the material weakening uniformly increases to the end (11) of the stent (1) or stent segment.

3. A stent according to Claim 1,
**characterised in that** the stent (1) is made from titanium, tantalum or another biocompatible metal.

4. A stent according to Claim 1,
charcterised in that a coating made from amorphous silicon carbide is provided.

## Revendications

1. Stent (1), en particulier stent coronaire, se composant d'au moins un élément tubulaire à paroi mince dont la surface enveloppe est réalisée avec des traversées en forme de filet et présente des cavités (3) qui sont limitées par des éléments (4) en forme de nervure de petite largeur, dans lequel les éléments (4) en forme de nervure ont été enlevés du matériau restant dans la zone des cavités (3) et dans lequel, vers l'extrémité (11) d'un stent (1), une résistance des éléments (4) en forme de nervure ou des zones de liaison adjacentes, formées par le matériau restant, à l'encontre de la déformation et de la déformation de rappel, est abaissée en comparaison au reste de la longueur du stent ou du segment de stent de sorte que sa rigidité, et de ce fait sa capacité d'avoir un effet d'expansion ou de maintenir une expansion appliquée sur le tissu, est diminuée et la réduction de la résistance à l'encontre de la déformation et de la déformation de rappel est provoquée par un affaiblissement du matériau, **caractérisé en ce que** la réduction de résistance à l'encontre de la déformation et de la déformation de rappel augmente vers l'extrémité (11) du stent (1).

2. Stent selon la revendication 1, **caractérisé en ce que** l'affaiblissement du matériau augmente régulièrement vers l'extrémité (11) du stent (1) ou du segment de stent.

3. Stent selon la revendication 1, **caractérisé en ce que** le stent (1) se compose de titane, de tantale ou d'un autre métal biocompatible.

4. Stent selon la revendication 1, **caractérisé en ce qu'**il est prévu un revêtement en carbure de silicium amorphe.
